# EUROPÄISCHE PATENTSCHRIFT

(11) **EP 2 968 271 B1**
(45) Veröffentlichungstag und Bekanntmachung des Hinweises auf die Patenterteilung: **09.01.2019**
(21) Anmeldenummer: 14722084.2
(22) Anmeldetag: 12.03.2014
(51) Int. Cl.: A61K 31/403, C07D 209/80, C07D 209/86, C07D 209/94, A61K 31/404, A61P 31/04

(54) **DERIVATE VON INDOLEN UND CARBAZOLEN, VERFAHREN ZU IHRER HERSTELLUNG SOWIE DEREN VERWENDUNG ALS MRSA-SENSITIVE ANTIBIOTIKA**
INDOLE AND CARBAZOLE DERIVATIVES, PROCESS FOR THEIR PREPARATION, AND THEIR USE AS MRSA SENSITIVE ANTIBIOTICS
DERIVÉS D'INDOLE ET DE CARBAZOLE, PROCÉDÉ POUR LEUR PRÉPARATION ET LEUR UTILISATION EN TANT QU'ANTIBIOTIQUES SENSIBLE AU MRSA

(30) Priorität: 13.03.2013 DE 102013004291
(43) Veröffentlichungstag der Anmeldung: 20.01.2016
(73) Patentinhaber: Martin-Luther-Universität Halle-Wittenberg, 06108 Halle/Saale (DE)
(72) Erfinder: HILGEROTH, Andreas, 06114 Halle (Saale) (DE)
(86) Internationale Anmeldenummer: PCT/DE2014/000142
(87) Internationale Veröffentlichungsnummer: WO 2014/139512

(56) Entgegenhaltungen:
- KATHERINE A. MCARTHUR ET AL: "Lynamicins A-E, Chlorinated Bisindole Pyrrole Antibiotics from a Novel Marine Actinomycete +", JOURNAL OF NATURAL PRODUCTS, Bd. 71, Nr. 10, 24. Oktober 2008 (2008-10-24), Seiten 1732-1737, XP055126526, ISSN: 0163-3864, DOI: 10.1021/np800286d
- Mardia El-Dessoky Teleb El-Sayed: "Development of Novel Indolyl-Derived Biologically Active Compounds", , 3. April 2013 (2013-04-03), Seiten 1-318, XP055126592, Gefunden im Internet: URL:http://digital.bibliothek.uni-halle.de /download/pdf/1643595?name=Development of novel indolyl-derived biologically active compounds [gefunden am 2014-07-03]

## Beschreibung

Staphylokokkus aureus (*S*. *aureus*) zählt zu den grampositiven Bakterien. Über Virulenzfaktoren kann *S. aureus* schwere Infektionen verursachen kann. Das sind z.T. lokale, oberflächliche Infektionen wie Wundinfektionen und Abzesse oder invasive, systemische Erkrankungen wie Osteomyelitis, Pneumonien, Endokarditis oder Sepsis [1-3]. Diese Infektionen sind besonders schwerwiegend bei immungeschwächten Patienten, werden aber auch in jüngster Zeit für immunkompetente Personen beschrieben [4]. Der Methicillinresistente *S. aureus* (MRSA) ist nicht nur - wie 90% der klinischen *S. aureus*-Isolate - gegen Penicilline resistent, sondern auch gegen Methicillin, das von dem die Penicilline spaltenden Enzym Penicillinase nicht gespalten wird [5,6]. Die Resistenz gegen Methicillin ist mit der Ausbildung eines zusätzlichen Penicillin-bindenden Proteins verbunden, das nur eine geringe Aktivität zu den β-Lactamantibiotika, zu denen auch das Methicillin gehört, aufweist [7]. Die Verbreitung von MRSA hat sowohl im Krankenhaus als auch außerhalb stark zugenommen. Von 1996 bis 2006 stieg der Anteil von MRSA von 8% um 12% auf 20% aller *S. aureus* Isolate [8]. Die Methicillin-Resistenz wird vom mecA-Gen codiert, das sich auf einer mobilen chromosomalen Kassette, der SCCmec-Kassette befindet, auf denen häufig auch Resistenzen gegen andere Antibiotika codiert sind, so die Resistenz gegen Bleomycin und Kanamycin oder gegen Erythromycin und Spectinomycin [9]. Somit sind viele MRSA-Stämme multiresistent gegen verschiedene Antibiotika bzw. Antibiotikaklassen. Als Reserveantibiotikum gg. MRSA galt lange Zeit das Vancomycin, ein Glykopeptid-Antibiotikum, gegen das jedoch auch zunehmend Resistenzen beschrieben werden, die von einem aus Enterokokken stammenden vanA-Gen hervorgerufen werden [10]. Auch gilt Vancomycin als problematisch, da seine Effektivität nicht hoch ist und es zudem toxisch ist [11]. Weitere Antibiotika-Alternativen sind das Oxazolidinon Linezolid oder die Streptogramine Quinopristin und Dalfopristin, deren Einsatz jedoch auch problematisch ist, teils aufgrund mangelnder Wirkeffektivitäten bei systemischen Erkrankungen oder wegen des Interaktionspotenzials, zum anderen auch aufgrund beschriebener Resistenzen [12,13]. Für das Glycylcyclin Tigecyclin wird jüngst eine erhöhte Mortalität beschrieben, die seine bereits beschränkte Anwendung für Haut- und Weichteilinfektionen weiter einschränkt [14].

Aufgabe der Erfindung ist die Entwicklung neuer Antibiotika, die mit neuartigen Strukturen und damit einem neuartigen Wirkprinzip antibiotisch wirksam sind. Bislang eingesetzte Antibiotika zeigen aufgrund ihrer Strukturen antibiotische Wirkprinzipien, gegen die die Bakterien genetisch über Gen(Plasmid)transfer Resistenzmechanismen entwickelt haben. Bei neuen Strukturen und damit neuen Wirkmechanismen ist eine Resistenz nicht gegeben und damit die Möglichkeit, resistente Keime erfolgreich zu bekämpfen.

Die erfindungsgemäß entwickelten 1,3-Bis(indol-3-yl)-tetrahydrocyclopenta[*b*]indole, 1,4-Bis(indol-3-yl)tetrahydrocarbazole, 6,10-Bis(indol-3-yl)hexahdrocyclohepta[*b*]indole, 6,11-Bis(indol-3-yl)hexahydrocycloocta[*b*]indole und 11-(Indol-3-yl)benzo[*b*]carbazole wirken selektiv hemmend auf das Wachstum von *S. aureus* und dem Methicillin-resistenten *S*. *aureus* (MRSA im unteren mikromolaren Konzentrationsbereich bei einem Screening an weiteren grampositiven und gramnegativen Bakterien und Pilzen.

Die Darstellung der Verbindungen aus Dialdehyden und Indolen bei Raumtemperatur in Eisessig stellt ein schonendes Verfahren dar, in dem die neuen Strukturen in teilweise sehr guten Ausbeuten zugänglich sind.

### Ausführungsbeispiele

1. Eine Stoffmenge von 2 mmol Malonaldehydbisdimethylacetal werden in 15 ml Eisessig unter Rühren bei Raumtemperatur gelöst. Hieraus entsteht der reaktive Malonaldehyd, zu dem 5 mmol des Indoles gegeben. Die klare, hellgelbe Lösung wird weiter über Nacht gerührt und nimmt dabei eine dunkelbraune Farbe an. Nach vier Wochen wird die Reaktionsmischung mit einer 10%igen wässrigen Natriumhydroxidlösung neutralisiert, wobei sich ein bräunlicher Niederschlag absetzt. Anschließend wird dreimal mit Dichlormethan extrahiert und die vereinten organischen Phasen werden zweimal mit Wasser und gesättigter Natriumchloridlösung gewaschen. Nach dem Trocknen über Natriumsulfat wird das Lösungsmittel im Vakuum abgezogen und der verbleibende Rückstand des 1,3-Bis(indol-3-yl)-tetrahydrocyclopenta[*b*]indoles wird säulenchromatographisch über Kieselgel mit Dichlormethan als Eluent gereinigt.
2. Eine Stoffmenge von 10 mmol 2,5-Dimethoxyfuran wird in 20 ml wässriger Salzsäure (10%) 2 h unter Rückfluss erhitzt. Nach dreimaliger Extraktion mit je 200 ml Diethylether wird die organische Phase mit Wasser und gesättigter Kochsalzlösung gewaschen und über Natriumsulfat getrocknet. Danach wird der Ether abdestilliert und 2 mmol des erhaltenen öligen Succinaldehydes werden in 15 ml Eisessig unter Rühren bei Raumtemperatur zusammen mit 5 mmol des Indols gelöst. Nach sechs Wochen Rühren bei Raumtemperatur wird die Reaktionsmischung mit einer 10%igen wässrigen Natriumhydroxidlösung neutralisiert, wobei sich ein bräunlicher Niederschlag absetzt. Anschließend wird dreimal mit Dichlormethan extrahiert und die vereinten organischen Phasen werden zweimal mit Wasser und gesättigter Natriumchloridlösung gewaschen. Nach dem Trocknen über Natriumsulfat wird das Lösungsmittel im Vakuum abgezogen und der verbleibende Rückstand des 1,4-Bis(indol-3-yl)tetrahydrocarbazoles wird säulenchromatographisch über Kieselgel mit Dichlormethan als Eluent gereinigt.
3. Eine Stoffmenge von 2 mmol Glutaraldehyd wird in 15 ml Eisessig gelöst und mit 5 mmol des Indols versetzt. Nach 8 Wochen Rühren bei Raumtemperatur wird die Reaktionsmischung mit einer 10%igen wässrigen Natriumhydroxidlösung neutralisiert, wobei sich ein bräunlicher Niederschlag absetzt. Anschließend wird dreimal mit Dichlormethan extrahiert und die vereinten organischen Phasen werden zweimal mit Wasser und gesättigter Natriumchloridlösung gewaschen. Nach dem Trocknen über Natriumsulfat wird das Lösungsmittel im Vakuum abgezogen und der verbleibende Rückstand des 6,10-Bis(indol-3-yl)hexahdrocyclohepta[*b*]indols wird säulenchromatographisch über Kieselgel mit Dichlormethan als Eluent gereinigt.
**4.** 10 mmol 7-Oxabicyclo[4.1.0]heptan werden in einer Lösung von 20 mmol Natriumperiodat in 40 ml wässriger THF-Lösung bei Raumtemperatur gerührt. Nach ermitteltem Abschluss der Reaktion mittels Dünnschichtchromatographie wird die wässrige Lösung filtriert und dreimal mit jeweils 30 ml Diethylether gewaschen. Die vereinigten Etherphasen werden mit Wasser und gesättigter Kochsalzlösung gewaschen, über Magnesiumsulfat getrocknet und konzentriert. Der Rückstand wird säulenchromatographisch aufgereingt. 2 mmol des erhaltenen öligen Adipaldehydes werden in 15 ml Eisessig gelöst und mit 5 mmol des Indols versetzt. Nach 10 Wochen Rühren bei Raumtemperatur wird die Reaktionsmischung mit einer 10%igen wässrigen Natriumhydroxidlösung neutralisiert, wobei sich ein bräunlicher Niederschlag absetzt. Anschließend wird dreimal mit Dichlormethan extrahiert und die vereinten organischen Phasen werden zweimal mit Wasser und gesättigter Natriumchloridlösung gewaschen. Nach dem Trocknen über Natriumsulfat wird das Lösungsmittel im Vakuum abgezogen und der verbleibende Rückstand des 6,11-Bis(indol-3-yl)hexahydrocycloocta[*b*]indols wird säulenchromatographisch über Kieselgel mit Dichlormethan als Eluent gereinigt.
5. 2 mmol o-Phthaladehyd werden in 15 ml Eisessig gelöst. Dazu werden 5 mmol des Indols gegeben. Nach mehrwöchigem Rühren bei Raumtemperatur wird der Abschluss der Reaktion mittels Dünnschichtchromatographie detektiert. Nach Neutralisation mit 10%iger Natriumhydroxidlösung wird dreimal mit jeweils 200 ml Dichlormethan extrahiert. Die vereinigten organischen Phasen werden mit Wasser und gesättigter Kochsalzlösung gewaschen, über Natriumsulfat getrocknet und nach Filtration im Vakuum konzentriert. Die Aufreinigung des 1-(Indol-3-yl)benzo[*b*]carbazoles erfolgt säulenchromatographisch über Kieselgel mit einer Lösung von Ethylacetat in Hexan (20%).

Zur Bestimmung der antimikrobiellen Aktivität werden die Substanzen jeweils in einer 12%igen wässrigen Lösung von DMSO in Wasser gelöst. Die Stammlösung von 200 µg/ml wird herunterverdünnt zu 50, 25, 12.5, 6.25 und 3.125 µg/ml in Mueller-Hinton Brühe und Sabouraud-Dextrose Brühe. Die Zellkulturen für die antibakterielle Testung wurden mit der Mueller-Hinton Brühe 24 Stunden bei 37 °C und für die antifungale Testung mit Sabouraud-Dextrose Brühe 24 Stunden bei 25 °C inkubiert. Die Testungen zur Bestimmung der MIC (minimal inhibitory concentration)-Werte erfolgten bei einem pH-Wert von 7.4. Die Inoculum-Größe für die antibakteriellen Tests betrug 10⁵ CFU/ml und für die antifungalen Tests 10⁴ CFU/ml. Die Inkubation mit den Substanzen erfolgte für 24 h bei 37 °C für die antibakterielle und für 48 Stunden bei 25 °C für die antifungale Aktivität. Hierbei zeigte ein 1,3-Bis(indol-3-yl)-tetrahydrocyclopenta[*b*]indol, ein 1,4-Bis(indol-3-ypl)tetrahydrocarbazol und ein 11-(Indol-3-yl)benzo[*b*]carbazol jeweils eine Wachstumshemmung bei einem MIC von 3.125 µg/ml in Bezug auf die Hemmung von *S*. *aureus* und MRSA. Ein 6,10-Bis(indol-3-yl)hexahydrocyclohepta[*b*]indol und ein 6,11-Bis(indol-3-yl)hexahydrocycloocta[*b*]indol zeigten eine Wachstumshemmung bei einem MIC von 3.125 µg/ml in Bezug auf die Hemmung von *S*. *aureus* und bei einem MIC von 6.25 µg/ml in Bezug auf die Hemmung von MRSA.

## Patentansprüche

1. Tetrahydrocyclopenta[*b*]indole, Tetrahydrocarbazole, Hexahydrocyclohepta[*b*]indole und Hexahydrocycloocta[*b*]indole der allgemeinen Formeln 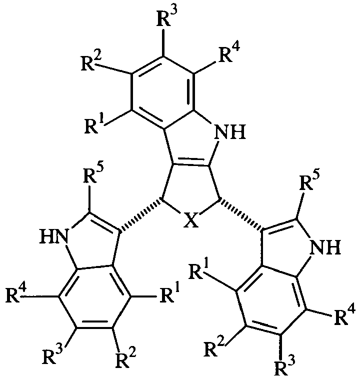
mit den Substitutionen X = CH-Br, -Cl, -OMe, -Me, -Pyridin-2-yl, Pyridin-4-yl, 4-Tolyl, 4-Anisyl, Pyrimidin-4-yl, 4-Chlorphenyl, CH(Y)-CH(Y), CH(Y)-CH₂, CH₂-CH(Y)-CH₂, CH(Y)-CH₂-CH₂, CH₂-CH₂-CH(Y)-CH₂ mit Y = OMe, Me, Br, Cl
mit den Resten
R¹ = H, NH₂, COOMe, NO₂, OMe, OH, OBn ;
R² = H, NH₂, NO₂, OMe, OH, Br, F, J, CN ;
R³ = H, COOMe, OMe, Br, F, OBn ;
R⁴ = H, NO₂, OMe, Cl, Br ;
R⁵ = H, Me, COOMe, -OEt ;

2. Funktionale 1,3-Bis(indol-3-yl)-tetrahydrocyclopenta[*b*]indole, 1,4-Bis(indol-3-yl)tetrahydrocarbazole, 6,10-Bis(indol-3-yl)hexahydrocyclohepta[b]indole, 6,11-Bis(indol-3-yl)hexahydrocycloocta[*b*]indole und 11-(Indol-3-yl)benzo[*b*]carbazole der allgemeinen Formeln 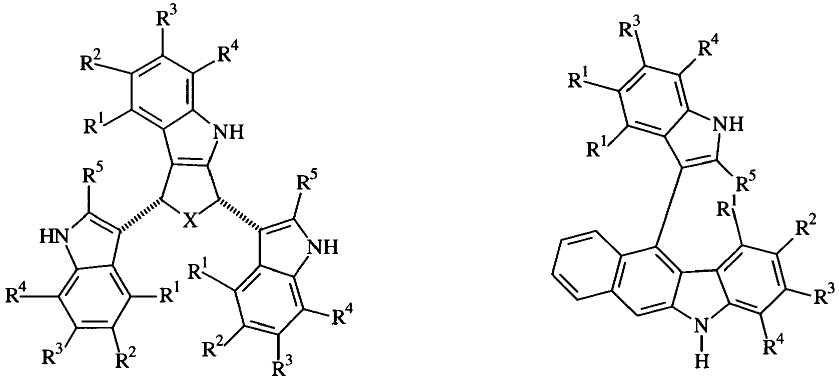
mit den Substitutionen X = CH-Br, -Cl, -OMe, -Me, -Pyridin-2-yl, Pyridin-4-yl, 4-Tolyl, 4-Anisyl, Pyrimidin-4-yl, 4-Chlorphenyl, CH(Y)-CH(Y), CH(Y)-CH₂, CH₂-CH(Y)-CH₂, CH(Y)-CH₂-CH₂, CH₂-CH₂-CH(Y)-CH₂ mit Y = OMe, Me, Br, Cl
mit den Resten
R¹ = H, NH₂, COOMe, NO₂, OMe, OH, OBn ;
R² = H, NH₂, NO₂, OMe, OH, Br, F, J, CN ;
R³ = H, COOMe, OMe, Br, F, OBn ;
R⁴ = H, NO₂, OMe, Cl, Br ;
R⁵ = H, Me, COOMe, -OEt ;
zur Herstellung MRSA-sensitiver Antibiotika.

3. Verbindungen der allgemeinen Formeln nach Anspruch 1 und 2, **gekennzeichnet dadurch, dass** diese als Wachstumshemmer gegen MRSA verwendet werden können.

4. Verfahren zur Herstellung der Verbindungen der allgemeinen Formeln nach Anspruch 1 und 2, **gekennzeichnet dadurch, dass** diese aus Indol und substituierten Indolen und Dialdehyden in Eisessig bei Raumtemperatur hergestellt werden.

## Claims

1. Tetrahydrocyclopenta[*b*]indole, Tetrahydrocarbazole, Hexahydrocyclohepta[*b*]indole and Hexahydrocycloocta[*b*]indole, of the general formulas 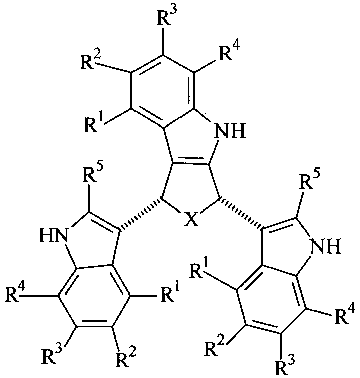
containing the substitutions X = CH-Br, -Cl, -OMe, -Me, -Pyridin-2-yl, Pyridin-4-yl, 4-Tolyl, 4- Anisyl, Pyrimidin-4-yl, 4-Chlorphenyl, CH(Y)-CH(Y), CH(Y)-CH₂, CH₂-CH(Y)-CH₂, CH(Y)-CH₂-CH₂, CH₂-CH₂-CH(Y)-CH₂ containing Y = OMe, Me, Br, Cl
containing the following residues
R¹ = H, NH₂, COOMe, NO₂, OMe, OH, OBn ;
R² = H, NH₂, COOMe, NO₂, OMe, OH, OBn ;
R³ = H, COOMe, OMe, Br, F, OBn ;
R⁴ = H, NO₂, OMe, Cl, Br ;
R⁵ = H, Me, COOMe, -OEt ;

2. Functional 1,3-Bis(indol-3-yl)-tetrahydrocyclopenta[*b*]indole, 1,4-Bis(indol-3-yl)tetrahydrocarbazole, 6,10-Bis(indol-3-yl)hexahydrocyclohepta[*b*]indole, 6,11-Bis(indol-3-yl)hexahydrocycloocta[*b*]indole und 11-(Indol-3-yl)benzo[*b*]carbazole, of the general formulas 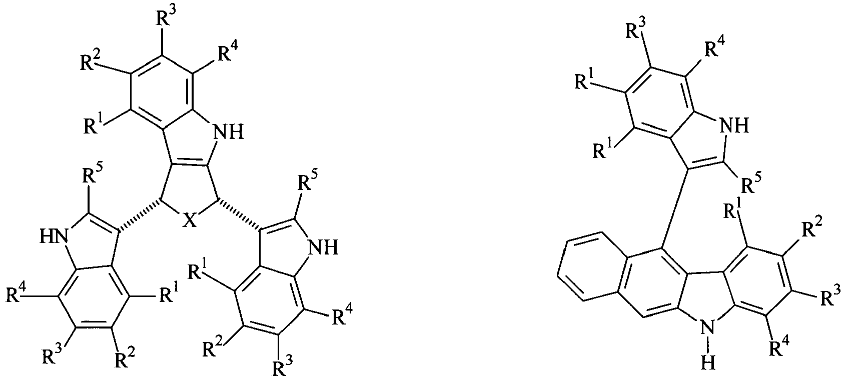
containing the substitutions X = CH-Br, -Cl, -OMe, -Me, -Pyridin-2-yl, Pyridin-4-yl, 4-Tolyl, 4- Anisyl, Pyrimidin-4-yl, 4-Chlorphenyl, CH(Y)-CH(Y), CH(Y)-CH₂, CH₂-CH(Y)-CH₂, CH(Y)-CH₂-CH₂, CH₂-CH₂-CH(Y)-CH₂ containing Y = OMe, Me, Br, Cl
containing the following residues
R¹ = H, NH₂, COOMe, NO₂, OMe, OH, OBn ;
R² = H, NH₂, NO₂, OMe, OH, Br, F, J, CN ;
R³ = H, COOMe, OMe, Br, F, OBn ;
R⁴ = H, NO₂, OMe, Cl, Br ;
R⁵ = H, Me, COOMe, -OEt;
for the production of MRSA sensitive antibiotics.

3. Compounds of the general formulas under requirement 1 and 2, **characterised by** the fact that they can be used to inhibit the growth of MRSA.

4. Process for producing the compounds of the general formulas under requirement 1 and 2, **characterised by** the fact that they can be produced from indoles and dialdehydes at room temperature in glacial acetic acid.

## Revendications

1. Tétrahydrocyclopenta[*b*]indoles, tétrahydrocarbazoles, héxahydrocyclohepta[*b*]indoles et héxahydrocycloocta[*b*]indoles des formules générales 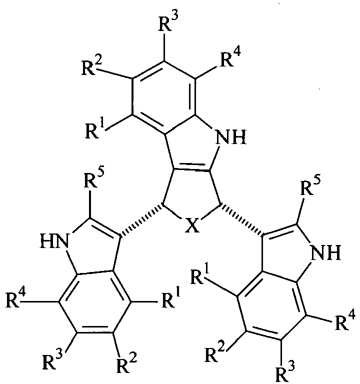
avec les substitutions X = CH-Br, -Cl, -OMe, -Me, -pyridine-2-yl, pyridine-4-yl, 4-tolyle, 4-anisyle, pyrimidine-4-yl, 4-chlorophényle, CH(Y)-CH(Y), CH(Y)-CH₂, CH₂-CH(Y)-CH₂, CH(Y)-CH₂-CH₂, CH₂-CH₂-CH(Y)-CH₂ avec Y = OMe, Me, Br, Cl
avec les résidus
R¹ = H, NH₂, COOMe, NO₂, OMe, OH, OBn ;
R² = H, NH₂, NO₂, OMe, OH, Br, F, J, CN ;
R³ = H, COOMe, OMe, Br, F, OBn ;
R⁴ = H, NO₂, OMe, Cl, Br ;
R⁵ = H, Me, COOMe, -OEt ;

2. 1,3-bis(indole-3-yl)-tétrahydrocyclopenta[*b*]indoles, 1,4-bis(indole-3-yl) tétrahydrocarbazoles, 6,10-bis(indole-3-yl)héxahydrocyclohepta[*b*]indoles, 6,11-bis(indole-3-yl)héxahydrocycloocta[*b*]indoles et 11-(indole-3-yl)benzo[*b*]carbazoles fonctionnels des formules générales 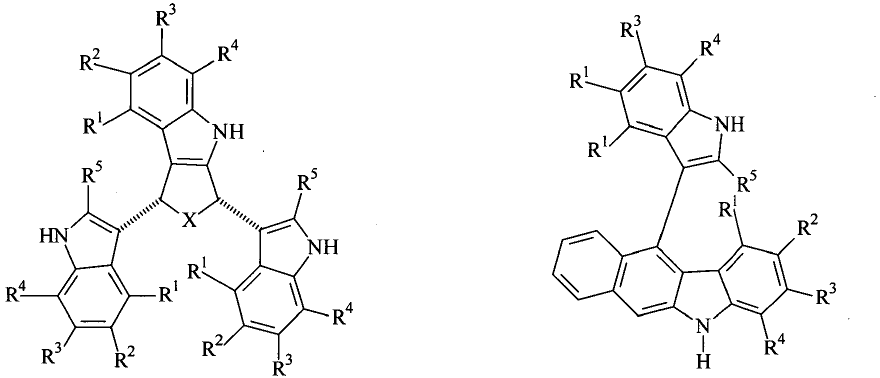
avec les substitutions X = CH-Br, -Cl, -OMe, -Me, -pyridine-2-yl, pyridine-4-yl, 4-tolyle, 4-anisyle, pyrimidine-4-yl, 4-chlorophényle, CH(Y)-CH(Y), CH(Y)-CH₂, CH₂-CH(Y)-CH₂, CH(Y)-CH₂-CH₂, CH₂-CH₂-CH(Y)-CH₂ avec Y = OMe, Me, Br, Cl
avec les résidus
R¹ = H, NH₂, COOMe, NO₂, OMe, OH, OBn ;
R² = H, NH₂, NO₂, OMe, OH, Br, F, J, CN ;
R³ = H, COOMe, OMe, Br, F, OBn ;
R⁴ = H, NO₂, OMe, Cl, Br ;
R⁵ = H, Me, COOMe, -OEt ;
en vue de la fabrication d'antibiotiques sensibles aux SARM.

3. Composés des formules générales selon les revendications 1 et 2, **caractérisées en ce qu'**ils peuvent servir d'inhibiteurs de croissance des SARM.

4. Procédé de fabrication des composés des formules générales selon les revendications 1 et 2, **caractérisés en ce qu'**ils sont fabriqués à partir d'indole et d'indoles et de dialdéhydes substitués dans de l'acide acétique glacial à température ambiante.
